# FASCICULE DE BREVET EUROPEEN

(11) **EP 1 248 651 B1**
(45) Date de publication et mention de la délivrance du brevet: **17.08.2005**
(21) Numéro de dépôt: 01903871.0
(22) Date de dépôt: 05.01.2001
(51) Int. Cl.: A61K 45/06, A61K 31/495, A61K 31/425, A61K 31/42, A61K 31/415, A61P 9/12

(54) **PRODUIT COMPRENANT UN INHIBITEUR DE LA TRANSDUCTION DES SIGNAUX DES PROTEINES G HETEROTRIMERIQUES EN ASSOCIATION AVEC UN AGENT ANTI-HYPERTENSEUR POUR UNE UTILISATION THERAPEUTIQUE DANS LE TRAITEMENT DE L'HYPERTENSION ARTERIELLE**
ZUSAMMENSETZUNG ENTHALTEND EINEN INHIBITOR DER SIGNALTRANSDUKTION VON HETEROMETRISCHEN G PROTEINEN IN KOMBINATION MIT EINEM ANTIHYPERTENSIVEN MITTEL ZU EINER THERAPEUTISCHEN VERWENDUNG IN DER BEHANDLUNG VON ARTERIELLER HYPERTONIE
PRODUCT COMPRISING A HETEROTRIMERIC G PROTEIN SIGNAL TRANSDUCTION INHIBITOR ASSOCIATED WITH ANTI-HYPERTENSIVE AGENT FOR THERAPEUTIC USE IN THE TREATMENT OF ARTERIAL HYPERTENSION

(30) Priorité: 06.01.2000 FR 0000105
(43) Date de publication de la demande: 16.10.2002
(73) Titulaire: SOCIETE DE CONSEILS DE RECHERCHES ET D'APPLICATIONS SCIENTIFIQUES (S.C.R.A.S.) Société par Actions Simplifiée, 75016 Paris (FR)
(72) Inventeur: PREVOST, Grégoire, F-92160 Antony (FR); TEILLOT, Marc Muséum National d'Histoire Naturelle, F-75005 Paris (FR)
(74) Mandataire: Bourgouin, André
(86) Numéro de dépôt international: PCT/FR2001/000027
(87) Numéro de publication internationale: WO 2001/049322

(56) Documents cités:
- WO-A-97/30053

## Description

La présente invention concerne un produit comprenant au moins un inhibiteur de protéine G, et de préférence un composé de formule générale **(I)** définie plus loin, en association avec au moins un agent anti-hypertenseur, de préférence choisi parmi le groupe composé des antagonistes des canaux calciques ou des inhibiteurs de l'enzyme de conversion, pour une utilisation thérapeutique simultanée, séparée ou étalée dans le temps, dans le traitement de l'hypertension artérielle.

L'hypertension artérielle est une affection dont la fréquence est considérable et qui est associée une morbidité et une mortalité élevée. Suivant l'âge, le traitement de l'hypertension doit être considéré quand la pression artérielle systolique est supérieure à 160-180 mm de mercure et la pression diastolique supérieure à 100-110 mm de mercure.

La stratégie optimale pour la prise en charge des hypertendus est encore en discussion. Le traitement non pharmacologique (réduction de l'apport alimentaire en sodium, perte de poids, exercice physique, arrêt du tabac...) représente une possibilité chez les sujets porteurs d'une hypertension modérée. Le traitement pharmacologique débute par une monothérapie qui permet un contrôle tensionnel satisfaisant chez 50-60% des patients. Le changement de classe thérapeutique ainsi que l'association avec une autre classe d'anti-hypertenseurs représentent les alternatives de traitements en cas de résistance à la première thérapie (Beaufils et Clément, *Drugs*, **56**, 11-21, (1998)).

Les médicaments anti-hypertenseurs se répartissent en plusieurs classes :
- les diurétiques thiazidiques et apparentés comme l'hydrochlorothiazide, le ciclétanine, le xipamide, l'indapamide et le clopamide ;
- les diurétiques de l'anse comme le furosémide, le pirétanide et le bumétanide ;
- les diurétiques hyperkaliémiants (épargnant le potassium) comme l'amiloride, la spironolactone et la canrénone ;
- les bêtabloquants comme le propranolol, l'acébutolol, l'aténolol, le nadolol, le bisoprolol, le métoprolol, le pindolol, l'oxprénolol et le bêtaxolol ;
- les inhibiteurs de l'enzyme de conversion (IEC) comme le captopril, l'énalapril, le bénazépril, le lisinopril, le quinapril, le ramipril et l'imidapril ;
- les antagonistes des récepteurs de l'angiotensine II (ARBs), comme le losartan, le candesartan, le cilexétil, l'irbesartan, le telmisartan, et le valsartan ;
- les antagonistes du canal calcique lent, comme la nifédipine, l'amlodipine, la félodipine, l'isradipine, le diltiazem, le bépridil, la lacidipine, la nitrendipine, la nicardipine et le vérapamil ;
- les anti-hypertenseurs centraux comme la clonidine, la guanfacine, la monoxidine, la rilménidine et l'α-méthyl-dopa ;
- les alphabloquants comme le prasozine, l'urapidil, la doxazosine et la térazosine ;
- les vasodilatateurs comme l'hydralazine, la dihydralazine et le minoxidil.

Par ailleurs, le développement des nouveaux traitements anti-hypertenseurs passe bien sûr par la découverte de nouvelles molécules (cf. Singh et al, *Drugs,* **58**(4):579-87 (Oct. 1999) ; (Mancia et al, *Curr. Opin. Cardiol.,* **14**(5):375-80 (Sep. 1999) ; Cases, *Drug new Perspect.,* **12**(6) 372-377 (1999)). Parmi les nouvelles familles de molécules destinées au traitement de l'hypertension, on peut notamment citer :
- les inhibiteurs de vasopeptidase ; et
- les antagonistes de l'endothéline.

Plus récemment, des variants alléliques des gènes codant pour l'angiotensinogène, le récepteur β2-adrénergique, et la sous-unité β3 de la protéine G ont été identifiés. (Luft, F.C., *Journal of hypertension,* **16**, 1871-1878, (1998)). De plus, les résultats de Anand-Srivastava (*Mol. Cell. Biochem.,* **175**, 163-170, (1996)) suggèrent que l'augmentation de l'expression des protéines Gi α-2 et Gi α-3 dans le coeur et l'aorte qui précède le développement de l'augmentation de la pression artérielle chez le rat SHR (*Spontaneously Hypertensive Rat* ou Rat Spontanément Hypertendu) peut être un des facteurs causant l'hypertension. De même, les sous-unités βγ de la protéine G semblent impliquer dans le contrôle de la resténose et la prolifération des cellules de muscles lisses vasculaires (Iaccarino et coll., *Proc. Nat. Acad. Sci. USA, 96,* 3945-3950,(1999)). Le blocage par un peptide βARKct de ces sous unités prévient la prolifération. D'autre part, un inhibiteur de la protéine Gi, la toxine pertussique, est capable de faire baisser la pression artérielle du rat SHR par une injection intraveineuse. Cette baisse de tension est observée pendant deux semaines et semble plus marquée chez le rat hypertendu que chez le rat normo-tendu (Kost et coll, Clinical and Experimental Pharmacology and Physiology, 26, 449-455, (1999)). De même le tonus rénal vasculaire chez le rat SHR est modifié par la toxine pertussique avec une augmentation du flux rénal vasculaire et une diminution de la résistance rénale vasculaire.

L'ensemble de ces travaux récents suggèrent que la protéine G hérérotrimérique peut représenter une cible thérapeutique pour le contrôle de l'hypertension par le développement de produits visant spécifiquement ce signal de transduction. Les protéines G participent à la transmission de signaux de l'extérieur de la cellule grâce à son interaction avec les récepteurs à sept domaines transmembranaires vers l'intérieur par l'intermédiaire de différents effecteurs incluant l'adénylate cyclase, la phospholipase C ou encore les canaux ioniques (cf. Gilman, A.G., *Biosci. Rep.,* **15**, 65-97 (1995)).

La demanderesse a d'ailleurs décrit des inhibiteurs spécifiques de la transduction du signal par les protéines G hétérotrimériques dans les demandes de brevet PCT WO 00/02558 (laquelle décrit l'utilisation des composés de formule générale **(I)** ci-après, déjà connus comme inhibiteurs de farnésyltransférases (cf. WO 97/30053), en tant qu'inhibiteurs de la transduction des signaux des protéines G hétérotrimériques) et WO 00/02881.

Quand la limite d'efficacité de la monothérapie est atteinte, la découverte d'associations efficaces entre différentes classes thérapeutiques est recherchée pour combiner l'effet de chaque classe et mieux combattre une hypertension d'origine multifactorielle. Par exemple, une association utilisant des bêtabloquants et des antagonistes calciques est efficace dans 80 à 85% des cas. Cette association permet de réduire les doses par rapport aux doses utilisées en monothérapie.

La demanderesse montre dans la présente demande que l'association d'un inhibiteur de protéine G avec un autre agent anti-hypertenseur, de préférence un agent anti-hypertenseur d'une autre classe, permet de réduire plus efficacement l'hypertension. Un produit selon l'invention offre l'avantage de pouvoir utiliser des doses moins élevées des agents anti-hypertenseurs choisis, ce qui a pour principal effet de diminuer les effets secondaires du traitement tout en obtenant un bénéfice thérapeutique équivalent.

L'invention a donc pour objet un produit comprenant au moins un inhibiteur de la transduction des signaux des protéines G hétérotrimériques en association avec au moins un agent anti-hypertenseur, de préférence un agent anti-hypertenseur d'une autre classe, ledit agent anti-hypertenseur étant de préférence choisi parmi le groupe composé des inhibiteurs des canaux calciques et des inhibiteurs de l'enzyme de conversion pour une utilisation thérapeutique simultanée, séparée ou étalée dans le temps, dans le traitement de l'hypertension artérielle.

De préférence, un produit selon l'invention est un Produit comprenant au moins un composé répondant à la formule générale **(I)** 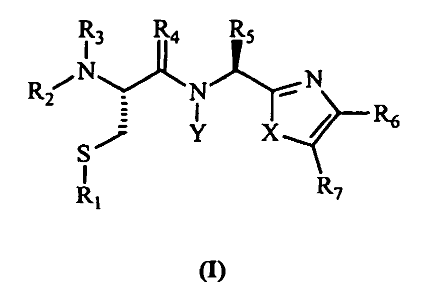 correspondant aux sous-formules **(I**_{**A**}**)** ou **(I**_{**B**}**)** : 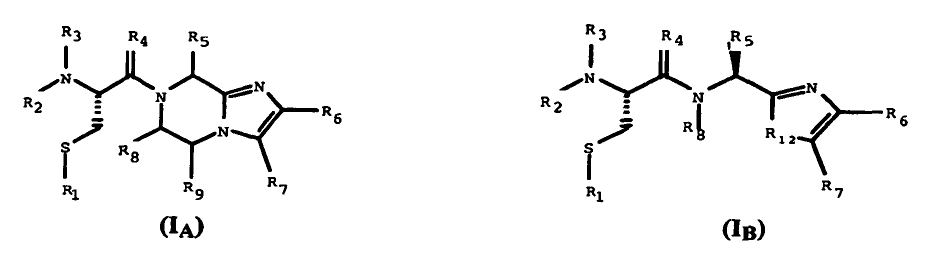 dans lesquelles :
X représente R₁₂ et Y représente R₈, ou X et Y complètent un cycle à 6 chaînons, l'ensemble X-Y représentant le radical -CH(R₈)-CH(R₉)- ;
R₁ représente H, un radical alkyle ou alkylthio comptant de 1 à 6 atomes de carbone ;
R₂ et R₃ représentent indépendamment H ou un radical alkyle comptant de 1 à 6 atomes de carbone ;
R₄ représente H₂ ou O ;
R₅ représente H, ou l'un des radicaux alkyle comptant de 1 à 6 atomes de carbone, alkényle comptant jusqu'à 6 atomes de carbone, alkynyle comptant jusqu'à 6 atomes de carbone, cycloalkyle, cycloalkylalkyle dont le radical alkyle compte de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé d'un radical alkyle comptant de 1 à 6 atomes de carbone, -O-R₁₀, -S(O)ₘR₁₀ (m représentant 0, 1, ou 2), -N(R₁₀)(R₁₁), -N-C(O)-R₁₀, -NH-(SO₂)-R₁₀, -CO₂-R₁₀, C(O)-N(R₁₀)(R₁₁), et -(SO₂)-N(R₁₀)(R₁₁) ;
R₆ et R₇ représentent indépendamment H, un radical -C(O)-NH-CHR₁₃-CO₂R₁₄, ou l'un des radicaux alkyle comptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁), et halo,
   ou R₆ et R₇ forment ensemble un radical aryle ou un hétérocycle ;
R₈ et R₉ représentent indépendamment, H, ou l'un des radicaux alkyle comptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁) et halo, ou R₈ et R₉ forment ensemble un radical aryle ou un hétérocycle ;
R₁₀ et R₁₁, représentent indépendamment H, un radical aryle ou hétérocycle, ou un radical alkyle comptant de 1 à 6 atomes de carbone, arylalkyle ou hétérocycle alkyle dont le radical alkyle compte de 1 à 6 atomes de carbone ;
R₁₂ représente NR₉, S, ou O ;
R₁₃ représente un radical alkyle comptant de 1 à 6 atomes de carbone éventuellement substitué par un radical choisi parmi les radicaux alkyle comptant de 1 à 6 atomes de carbone, -OR₁₀, -S(O)ₘR₁₀ (m représentant 0, 1, ou 2) et -N(R₁₀)(R₁₁);
R₁₄ représente H ou un radical alkyle comptant de 1 à 6 atomes de carbone ;
le composé de formule générale **(I)** pouvant le cas échéant aussi se présenter sous la forme dimère d'un disulfure ;
ou un sel pharmaceutiquement acceptable d'un composé de formule générale (1) ou le cas échéant de son dimère ;
et au moins un autre agent anti-hypertenseur choisi parmi le groupe constitué par les inhibiteurs de canaux calciques, les inhibiteurs d'enzyme de conversion, les diurétiques thiazidiques et apparentés, les diurétiques de l'anse, les diurétiques épargnant le potassium, les antialdostérones, les bêtabloquants, les antagonistes des récepteurs de l'angiotensine, les anti-hypertenseurs centraux, les alpha-bloquants et vasodilatateurs, les inhibiteurs de vasopeptidase et les antagonistes de l'endothéline pour une utilisation thérapeutique simultanée, séparée ou étalée dans le temps, dans le traitement de l'hypertension artérielle.

Par radical alkyle inférieur, on entend un radical alkyle linéaire ou ramifié comptant de 1 à 6 atomes de carbone, et notamment les radicaux méthyle, éthyle, propyle, isopropyle, butyle, isobutyle, sec-butyle et tert-butyle, pentyle, néopentyle, isopentyle, hexyle, isohexyle. Par radical hétérocycle, on entend un radical constitué de un ou plusieurs cycles et incluant au moins un hétéroatome (O, N ou S). Par radical aryle, on entend un système aromatique carbocyclique mono- ou polycyclique comprenant au moins un cycle aromatique (et, en particulier, le radical phényle qui peut être noté de façon abrégée Ph). Par radical arylalkyle, hétérocycle alkyle, alkylthio ou alkoxy inférieur, on entend les radicaux dont le radical alkyle a la signification indiquée précédemment.

De préférence, les composés de formule générale **(I)** seront tels que :
X et Y complètent un cycle à 6 chaînons, l'ensemble X-Y représentant le radical -CH(R₈)-CH(R₉)- ;
R₁ représente un radical alkyle comptant de 1 à 6 atomes de carbone;
R₂ et R₃ représentent H ;
R₄ représente O ;
R₅ représente H, ou l'un des radicaux alkyle comptant de 1 à 6 atomes de carbone cycloalkyle, cycloalkylalkyle, arylsulfonylalkyle dont le radical alkyle comptant de 1 à 6 atomes de carbone, aralkoxyalkyle dont le radical alkyle comptant de 1 à 6 atomes de carbone, ces radicaux pouvant éventuellement être substitués par des radicaux choisis parmi le groupe composé d'un radical alkyle inférieur ou -O-R₁₀ ;
R₆ et R₇ représentent indépendamment H ou un radical aryle éventuellement substitué par des radicaux choisis parmi le groupe composé des radicaux OH, alkyle ou alkoxy inférieur,
R₈ et R₉ représentent H ;
et R₁₀ et R₁₁, représentent indépendamment H ou un radical alkyle comptant de 1 à 6 atomes de carbone.

Sont en particulier préférés pour l'invention les composés de formule générale **(I)** suivants :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-butyl-2-(2-méthoxyphényl)-5,6,7,8-tétrahydroimidazo[1,2a]pyrazine ;
- le disulfure de bis-1,1'-[7-(2-amino-1-oxo-3-thiopropyl)-2-(2-méthoxyphényl)-8-(1-méthylpropyl)-5,6,7,8-tétrahydroimidazo[1,2a]pyrazine] ;
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) ;
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7 -(2-amino-1-oxo-3-tbiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydro-imidazo[1,2a]pyrazine, ou sa forme dimérique ;
- et la 7-(2-amino-1-oxo-3-thiopropyl)-2-(2-méthoxyphényl)-8-(phénylsulfonyléthyl)-5,6,7,8-tétrahydro-imidazo[1,2a]pyrazine ;
ou un sel pharmaceutiquement acceptable d'un de ces derniers.

En ce qui concerne l'agent anti-hypertenseur associé à l'inhibiteur de la transduction des signaux des protéines G hétérotrimériques, bien que les inhibiteurs de canaux calciques et les inhibiteurs d'enzyme de conversion, et en particulier le vérapamil et le captopril, soient préférés, de nombreux autres agents anti-hypertenseurs peuvent être également utilisés selon l'invention, tels que les diurétiques thiazidiques et apparentés, les diurétiques de l'anse, les diurétiques épargnant le potassium, les antialdostérones, les bêtabloquants, les antagonistes des récepteurs de l'angiotensine, les anti-hypertenseurs, les alpha-bloquants et vasodilatateurs, les inhibiteurs de vasopeptidase et les antagonistes de l'endothéline.

Selon une variante particulièrement préférée de l'invention, l'inhibiteur de la transduction des signaux des protéines G hétérotrimériques entrant dans la composition d'un produit selon l'invention sera choisi parmi les composés suivants :
- la 7-(2-amino-1-oxo-3-thiopropyl)-8-(cyclohexylméthyl)-2-phényl-5,6,7,8-tétrahydroimidazo[1,2a]pyrazine ; et
- la 7-(2-amino-1-oxo-3-thiopropyl)-8-(cyclohexylméthyl)-2-(2-méthylphényl)-5,6,7,8-tétrahydroimidazo[1,2a]pyrazine ;
et des sels pharmaceutiquement acceptables de ces derniers.

Toujours selon une variante particulièrement préférée de l'invention, les agents anti-hypertenseurs associés auxdits inhibiteurs de la transduction des signaux des protéines G hétérotrimériques seront choisis parmi le groupe composé :
- des antagonistes des canaux calciques, et en particulier le vérapamil ;
- des inhibiteurs de l'enzyme de conversion, et en particulier le captopril ;
et des sels pharmaceutiquement acceptables de ces derniers.

Optionnellement, un deuxième agent anti-hypertenseur, différent de l'inhibiteur de la transduction des signaux des protéines G hétérotrimériques et de l'agent anti-hypertenseur qui lui est déjà associé, pourra être associé à un produit selon l'invention. Ledit agent anti-hypertenseur pourra être choisi parmi ceux déjà cités dans la présente demande. Seront en particulier préférés les produits associant :
- un inhibiteur de la transduction des signaux des protéines G hétérotrimériques, un diurétique de l'anse et un diurétique hyperkaliémiant ;
- un inhibiteur de la transduction des signaux des protéines G hétérotrimériques, un diurétique thiazidique ou apparenté et un diurétique hyperkaliémiant ;
- un inhibiteur de la transduction des signaux des protéines G hétérotrimériques, un inhibiteur de l'enzyme de conversion et un diurétique thiazidique ;
- un inhibiteur de la transduction des signaux des protéines G hétérotrimériques, un antagoniste des récepteurs de l'angiotensine II et un diurétique thiazidique ;
- un inhibiteur de la transduction des signaux des protéines G hétérotrimériques, un bêtabloquant et un diurétique ;
- un inhibiteur de la transduction des signaux des protéines G hétérotrimériques, un bêtabloquant et un antagoniste du canal calcique ;
- un inhibiteur de la transduction des signaux des protéines G hétérotrimériques, un bêtabloquant et un vasodilatateur.

L'invention a également pour objet une composition pharmaceutique comprenant un produit selon l'invention, avec éventuellement un ou des excipients pharmaceutiquement acceptables.

Les compositions pharmaceutiques comprenant un composé de l'invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse, etc.), etc. La voie d'administration dépendra bien entendu du type de maladie à traiter.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

Les doses d'administration suivantes (journalières, sauf indication contraire) pourront notamment être envisagées pour les différents composés entrant dans la composition d'un produit selon l'invention :
- composé de formule générale **(I)** : de 0,1 à 100 mg/kg par voie intraveineuse ; et 50 à 1000 mg per os par jour en plusieurs prises
- vérapamil : 50 à 500 mg per os par jour en plusieurs prises ;
- captopril : 50 à 500 mg per os par jour en plusieurs prises.

Pour les autres composés entrant dans la composition des produits selon l'invention, les doses d'administration journalières seront fixées par le médecin ou le vétérinaire traitant dans la limite des doses de ces composés habituellement administrées pour le traitement de l'hypertension artérielle, lesquelles pourront notamment être trouvées dans un ouvrage de référence (comme, par exemple, le *dictionnaire VIDAL®*, la *Rote Liste®* ou le *Physician's Desk Reference*®).

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### Préparation des composés de formule générale (I) entrant dans la composition des produits de l'invention :

A) Les composés de formule générale **(I)** peuvent préparés selon des méthodes analogues à celles décrites dans la demande de brevet PCT WO 97/30053.
B) La préparation de certains composés particuliers de formule générale **(I),** non décrits dans la demande PCT WO 97/30053, est décrite dans la demande PCT WO 00/02881.

### EXEMPLES

Afin d'illustrer l'utilité de l'invention, on étudiera l'effet d'un traitement sur la tension artérielle de rats spontanément hypertendus par la 7-(2-amino-1-oxo-3-thiopropyl)-8-(cyclohexylméthyl)-2-phényl-5,6,7,8-tétrahydroimidazo[1,2a]pyrazine (ci-après désignée composé 2, décrite dans la demande WO 00/02881), en association avec des agents anti-hypertenseurs de deux classes distinctes, à savoir les inhibiteurs de l'enzyme de conversion (captopril) et les inhibiteurs des canaux calciques (vérapamil).

### 1) Procédures

Des rats mâles SHR de 13/14 semaines (Charles River France) sont anesthésiés à l'aide de pentobarbital (Sanofi) (60 mg/kg/IP). Une carotide est cathétèrisée pour la mesure de la pression artérielle et de la fréquence cardiaque (capteurs de pression et physiographe Gould, logiciel d'acquisition Buxco version 1.5.7 et logiciel d'analyse Analyst version 1.35 (EMKA)). Une jugulaire sera cathétérisée pour l'injection de l'anti-hypertenseur, le composé 2 sera lui injecté dans la veine du pénis. Après une période de stabilisation de 10 minutes, le produit ou les deux simultanément sont administrés et leur effet sont suivis durant 20 minutes.

Aux fins de leur administration, la 7-(2-amino-1-oxo-3-thiopropyl)-8-(cyclohexylméthyl)-2-phényl-5,6,7,8-tétrahydro-imidazo[1,2a]pyrazine, le vérapamil (Sigma, USA) et le captopril (Sigma, USA) sont solubilisés dans une solution aqueuse de NaCl à 0,9%.

Les groupes suivants (de 4 à 6 animaux) sont constitués :
- composé 2 (1 mg/kg) ;
- captopril (1 mg/kg) ;
- vérapamil (0,1 et 0,3 mg/kg) ;
- captopril (1 mg/kg) + composé 2 (1 mg/kg) ;
- vérapamil (0,1 ou 0,3 mg/kg) + composé 2 (1 mg/kg) ;

### 2) Résultats :

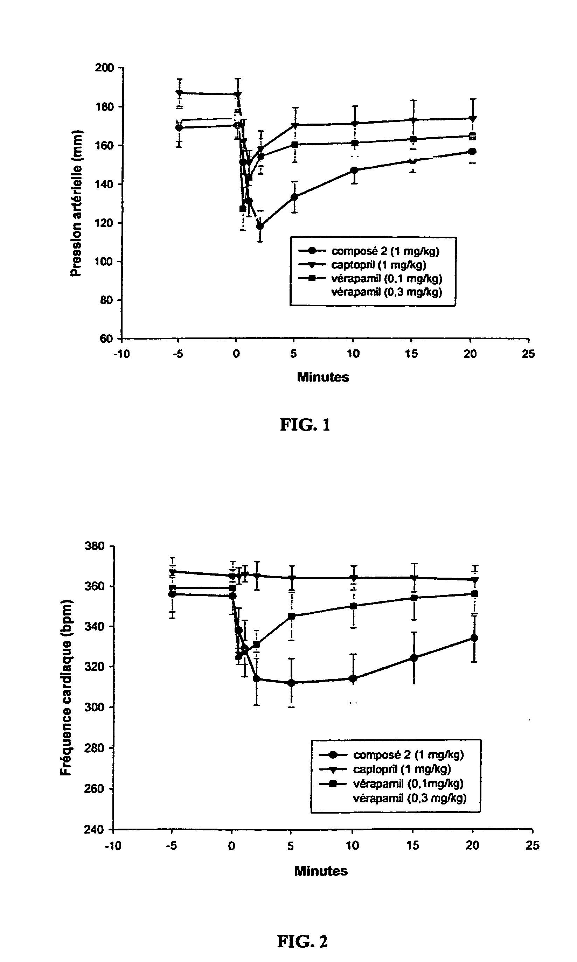

Les rats spontanément hypertendus anesthésiés présentent une tension artérielle moyenne variant entre 165-190 mm. (cf. Figures 1, 2 et 3).

Les traitements par une injection intraveineuse unique de l'inhibiteur des canaux calciques vérapamil (0,1 mg/kg ou 0,3 mg/kg), ou de l'inhibiteur de l'enzyme de conversion captopril (1 mg/kg) ou de l'inhibiteur de la protéine G hétérotrimérique (1 mg/kg) entraînent une diminution immédiate (moins de 2 minutes) de la pression artérielle avec un retour aux valeurs initiales après 20 minutes après les injections faites au temps 0 (Figure 1).

Par ailleurs, les fréquences cardiaques mesurées sur ces rats SHR anesthésiés avant injection se situent entre 328 et 371 battements par minutes. Les fréquences cardiaques mesurées sur ces rats SHR traités par les mêmes produits, représentées sur la Figure 2, sont très peu modifiées par ces différents traitements. Le captopril ne modifie pas du tout la fréquence, alors que le vérapamil (0,1 et 0,3 mg/kg) ou l'inhibiteur de protéine G (1 mg/kg) induisent une diminution avec une différence (delta) maximale de 32, 56 et 44 respectivement.

L'association de plusieurs anti-hypertenseurs est fréquemment prescrite pour obtenir une stabilisation de la tension artérielle.

L'association entre un inhibiteur de la protéine G et deux autres classes thérapeutiques est illustrée ci après.

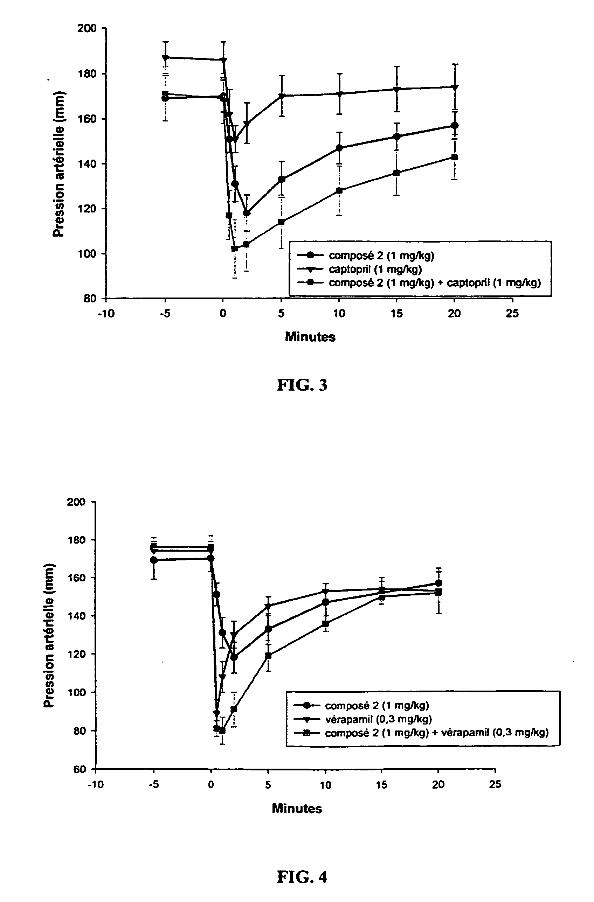

La Figure 3 montre que l'association « inhibiteur de protéine G + captopril » induit une diminution de la pression artérielle chez le rat SHR supérieure à l'activité des deux produits seuls.

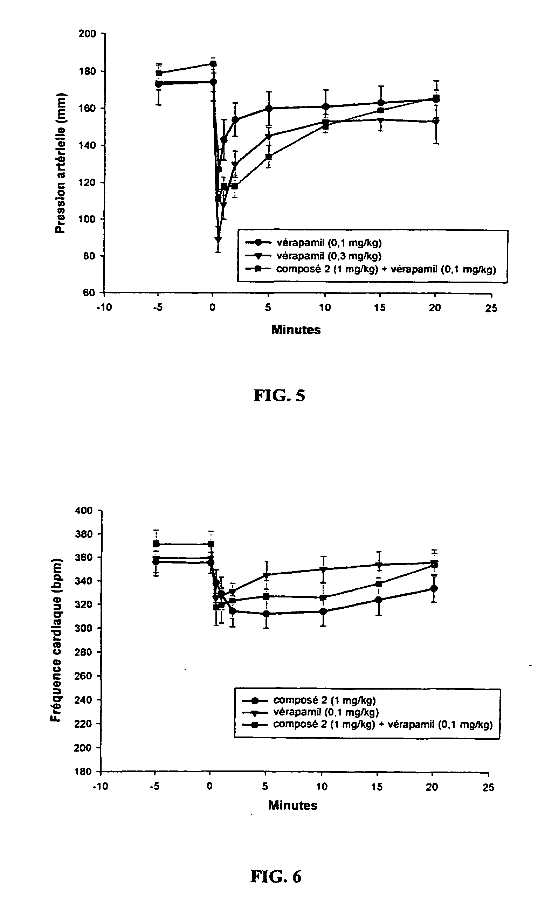

Par contre la figure 4 montre que l'association inhibiteur de protéine G + captopril n'induit pas une diminution de la fréquence cardiaque chez le rat SHR supérieure à l'activité des deux produits seuls.

La figure 5 montre que l'association « inhibiteur de protéine G + vérapamil » induit une diminution de la pression artérielle chez le rat SHR supérieure à l'activité des deux produits seuls. L'association « inhibiteur de protéine G + vérapamil à la dose 0,1 mg/kg» donne un profil semblable à l'action du vérapamil seul, mais à une dose 3 fois supérieure soit 0,3 mg/kg (Figure 5).

Par contre, les résultats illustrés par la figure 6 et le tableau ci-après montrent que l'association « inhibiteur de protéine G + vérapamil » n'induit pas une diminution de la fréquence cardiaque chez le rat SHR supérieure à l'activité des deux produits seuls.

| **Produit** | **Dose(s) (mg/kg)** | **Différence de fréquence (Δ)** |
|---|---|---|
| Composé 2 | 1 | 44 |
| Captopril | 1 | 1 |
| Vérapamil | 0,1 | 28 |
| Vérapamil | 0,3 | 56 |
| Composé 2 + captopril | 1 et 1 | 25 |
| Composé 2 + vérapamil | 1 et 0,1 | 54 |
| Composé 2 + vérapamil | 1 et 0,3 | 52 |

## Revendications

1. Produit comprenant au moins un composé répondant à la formule générale **(I)** 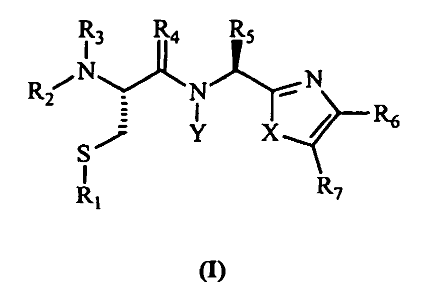 correspondant aux sous-formules **(I**_{**A**}**)** ou **(I**_{**B**}**)** : 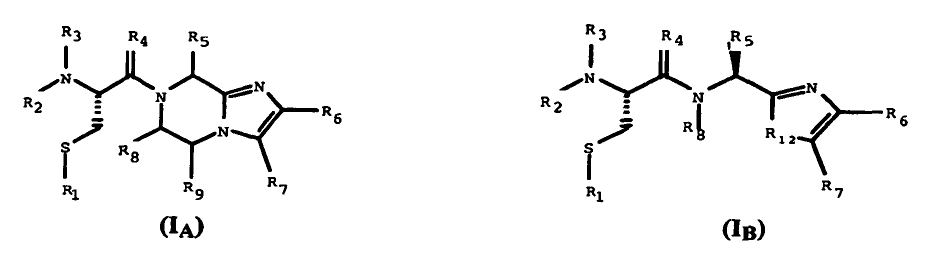 dans lesquelles :
X représente R₁₂ et Y représente R₈, ou X et Y complètent un cycle à 6 chaînons, l'ensemble X-Y représentant le radical -CH(R₈)-CH(R₉)- ;
R₁ représente H, un radical alkyle ou alkylthio comptant de 1 à 6 atomes de carbone ;
R₂ et R₃ représentent indépendamment H ou un radical alkyle comptant de 1 à 6 atomes de carbone ;
R₄ représente H₂ ou O ;
R₅ représente H, ou l'un des radicaux alkyle comptant de 1 à 6 atomes de carbone, alkényle comptant jusqu'à 6 atomes de carbone, alkynyle comptant jusqu'à 6 atomes de carbone, cycloalkyle, cycloalkylalkyle dont le radical alkyle compte de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé d'un radical alkyle comptant de 1 à 6 atomes de carbone, -O-R₁₀, -S(O)ₘR₁₀ (m représentant 0, 1, ou 2), -N(R₁₀)(R₁₁), -N-C(O)-R₁₀, -NH-(SO₂)-R₁₀, -CO₂-R₁₀, C(O)-N(R₁₀)(R₁₁), et -(SO₂)-N(R₁₀)(R₁₁) ;
R₆ et R₇ représentent indépendamment H, un radical -C(O)-NH-CHR₁₃-CO₂R₁₄, ou l'un des radicaux alkyle comptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁), et halo,
ou R₆ et R₇ forment ensemble un radical aryle ou un hétérocycle ;
R₈ et R₉ représentent indépendamment, H, ou l'un des radicaux alkyle comptant de 1 à 6 atomes de carbone, aryle, arylalkyle dont le radical alkyle compte de 1 à 6 atomes de carbone, hétérocycle ou hétérocycle alkyle dont le radical alkyle compte de 1 à 6 atomes de carbone, ces radicaux pouvant éventuellement être substitués par des radicaux choisis parmi le groupe composé des radicaux OH, alkyle ou alkoxy comptant de 1 à 6 atomes de carbone, N(R₁₀)(R₁₁), COOH, CON(R₁₀)(R₁₁) et halo, ou R₈ et R₉ forment ensemble un radical aryle ou un hétérocycle ;
R₁₀ et R₁₁, représentent indépendamment H, un radical aryle ou hétérocycle, ou un radical alkyle comptant de 1 à 6 atomes de carbone, arylalkyle ou hétérocycle alkyle dont le radical alkyle compte de 1 à 6 atomes de carbone ;
R₁₂ représente NR₉, S, ou O ;
R₁₃ représente un radical alkyle comptant de 1 à 6 atomes de carbone éventuellement substitué par un radical choisi parmi les radicaux alkyle comptant de 1 à 6 atomes de carbone, -OR₁₀, -S(O)ₘR₁₀ (m représentant 0, 1, ou 2) et -N(R₁₀)(R₁₁);
R₁₄ représente H ou un radical alkyle comptant de 1 à 6 atomes de carbone ;
le composé de formule générale **(I)** pouvant le cas échéant aussi se présenter sous la forme dimère d'un disulfure ;
ou un sel pharmaceutiquement acceptable d'un composé de formule générale (1) ou le cas échéant de son dimère ;
et au moins un autre agent anti-hypertenseur choisi parmi le groupe constitué par les inhibiteurs de canaux calciques, les inhibiteurs d'enzyme de conversion, les diurétiques thiazidiques et apparentés, les diurétiques de l'anse, les diurétiques épargnant le potassium, les antialdostérones, les bêtabloquants, les antagonistes des récepteurs de l'angiotensine, les anti-hypertenseurs centraux, les alpha-bloquants et vasodilatateurs, les inhibiteurs de vasopeptidase et les antagonistes de l'endothéline pour une utilisation thérapeutique simultanée, séparée ou étalée dans le temps, dans le traitement de l'hypertension artérielle.

2. Produit selon la revendication 1, **caractérisé en ce que** le composé de formule générale **(I)** est tel que :
X et Y complètent un cycle à 6 chaînons, l'ensemble X-Y représentant le radical -CH(R₈)-CH(R₉)- ;
R₁ représente un radical alkyle comptant de 1 à 6 atomes de carbone ;
R₂ et R₃ représentent H ;
R₄ représente O ;
R₅ représente H, ou l'un des radicaux alkyle comptant de 1 à 6 atomes de carbone, cycloalkyle, cycloalkylalkyle dont le radical alkyle compte de 1 à 6 atomes de carbone, arylsulfonylalkyle dont le radical alkyle compte de 1 à 6 atomes de carbone, aralkoxyalkyle dont les radicaux alkoxy et alkyle comptent chacun de 1 à 6 atomes de carbone, ces radicaux pouvant éventuellement être substitués par des radicaux choisis parmi le groupe composé d'un radical alkyle comptant de 1 à 6 atomes de carbone ou -O-R₁₀ ;
R₆ et R₇ représentent indépendamment H ou un radical aryle éventuellement substitué par des radicaux choisis parmi le groupe composé des radicaux OH et alkyle ou alkoxy comptant de 1 à 6 atomes de carbone ;
R₈ et R₉ représentent H ;
et R₁₀ et R₁₁, représentent indépendamment H ou un radical alkyle comptant de 1 à 6 atomes de carbone.

3. Produit selon la revendication 1, **caractérisé en ce que** le composé de formule générale **(I)** est l'un des composés suivants :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la -7-(2-amino-1-oxo-3-thiopropyl)-8-butyl-2-(2-méthoxyphényl)-5,6,7,8-tétrahydroimidazo[1,2a]pyrazine ;
- le disulfure de bis-1,1'-[7-(2-amino-1-oxo-3-thiopropyl)-2-(2-méthoxyphényl)-8-(1-méthylpropyl)-5,6,7,8-tétrahydroimidazo[1,2a]pyrazine] ;
- le disulfure de bis-1,1'-[7-(2-amino-1-oxo-3-thiopropyl)-8-(cyclohexylméthyl)-2-(2-méthoxyphényl)-5,6,7,8-tétrahydroimidazo[1,2a]pyrazine ;
- le disulfure de bis-1,1'-7-(2-amino-1-oxo-3-thiopropyl-(2-(1-naphtyl)-8-(2-méthylpropyl)-5,6,7,8-tétrahydroimidazo[1,2a]pyrazin-7-yle) ;
- la 7-(2-amino-1-oxo-3-thiopropyl)-8-(cyclohexylméthyl)-2-phényl-5,6,7,8-tétrahydroimidazo[1,2a]pyrazine ;
- la 7-(2-amino-1-oxo-3-thiopropyl)-2-(2-méthoxyphényl)-8-(phénylméthoxy)méthyl-5,6,7,8-tétrahydroimidazo[1,2a]pyrazine ;
- la 7-(2-amino-1-oxo-3-thiopropyl)-2-(2-méthoxyphényl)-8-(1-phénylméthoxy)éthyl-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydroimidazo[1,2a]pyrazine ;
- la 7-(2-amino-1-oxo-3-thiopropyl)-2-(2-méthoxyphényl)-8-(phénoxyéthyl)-5,6,7,8-tétrahydro-imidazo[1,2a]pyrazine, ou sa forme dimérique ;
- et la 7-(2-amino-1-oxo-3-thiopropyl)-2-(2-méthoxyphényl)-8-(phénylsulfonyléthyl)-5,6,7,8-tétrahydro-imidazo[1,2a]pyrazine ;
ou un sel pharmaceutiquement acceptable d'un de ces derniers.

4. Produit selon la revendication 3, **caractérisé en ce que** le composé de formule générale **(I)** est la 7-(2-amino-1-oxo-3-thiopropyl)-8-(cyclohexylméthyl)-2-phényl-5,6,7,8-tétrahydro-imidazo[1,2a]pyrazine ou un sel pharmaceutiquement acceptable de cette dernière.

5. Produit selon l'une de revendications 1 à 4, **caractérisé en ce que** l'agent anti-hypertenseur associé à l'inhibiteur de la transduction des signaux des protéines G hétérotrimériques est un inhibiteur de canaux calciques.

6. Produit selon la revendication 5, **caractérisé en ce que** l'agent anti-hypertenseur associé à l'inhibiteur de la transduction des signaux des protéines G hétérotrimériques est le vérapamil.

7. Produit selon la revendication l'une des revendications 1 à 4, **caractérisé en ce que** l'agent anti-hypertenseur associé à l'inhibiteur de la transduction des signaux des protéines G hétérotrimériques est un inhibiteur d'enzyme de conversion.

8. Produit selon la revendication 7, **caractérisé en ce que** l'agent anti-hypertenseur associé à l'inhibiteur de la transduction des signaux des protéines G hétérotrimériques est le captopril.

9. Produit selon l'une des revendications précédentes, **caractérisé en ce qu'**il comprend en outre un deuxième agent anti-hypertenseur, celui-ci étant différent du composé de formule générale **(I)** et de l'agent anti-hypertenseur qui lui est déjà associé.

10. Produit selon la revendication 9, **caractérisé en ce que** le premier agent antihypertenseur associé au composé de formule générale **(I)** est un diurétique de l'anse et le deuxième un diurétique hyperkaliémiant.

11. Produit selon la revendication 9, **caractérisé en ce que** le premier agent antihypertenseur associé au composé de formule générale **(I)** est un diurétique thiazidique ou apparenté et le deuxième un diurétique hyperkaliémiant.

12. Produit selon la revendication 9, **caractérisé en ce que** le premier agent antihypertenseur associé au composé de formule générale (I) est un inhibiteur de l'enzyme de conversion et le deuxième un diurétique thiazidique.

13. Produit selon la revendication 9, **caractérisé en ce que** le premier agent antihypertenseur associé au composé de formule générale **(I)** est un antagoniste des récepteurs de l'angiotensine II et le deuxième un diurétique thiazidique.

14. Produit selon la revendication 9, **caractérisé en ce que** le premier agent antihypertenseur associé au composé de formule générale **(I)** est un bêtabloquant et le deuxième un diurétique.

15. Produit selon la revendication 9, **caractérisé en ce que** le premier agent antihypertenseur associé au composé de formule générale **(I)** est un bêtabloquant et le deuxième un antagoniste du canal calcique.

16. Produit selon la revendication 9, **caractérisé en ce que** le premier agent antihypertenseur associé à l'inhibiteur de la transduction des signaux des protéines G hétérotrimériques est un bêtabloquant et le deuxième un vasodilatateur.

17. Composition pharmaceutique comprenant au moins un composé de formule générale **(I)** tel que défini dans la revendication 1 et au moins un autre agent anti-hypertenseur choisi parmi le groupe constitué par les inhibiteurs de canaux calciques, les inhibiteurs d'enzyme de conversion, les diurétiques thiazidiques et apparentés, les diurétiques de l'anse, les diurétiques épargnant le potassium, les antialdostérones, les bêtabloquants, les antagonistes des récepteurs de l'angiotensine, les anti-hypertenseurs, les alpha-bloquants et vasodilatateurs, les inhibiteurs de vasopeptidase et les antagonistes de l'endothéline, avec éventuellement un ou des excipients pharmaceutiquement acceptables.

## Claims

1. Product comprising at least one compound corresponding to general formula (I) 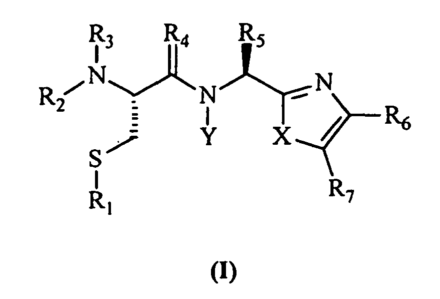 corresponding to sub-formulae **(I**_{**A**}**)** or **(I**_{**B**}**):** 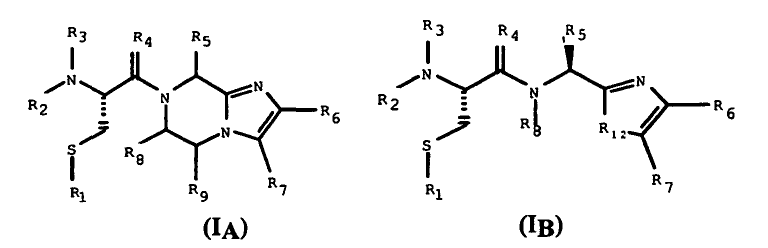 in which:
X represents R₁₂ and Y represents R₈, or X and Y complete a ring with 6 members, X-Y together representing the -CH(R₈)-CH(R₉)- radical;
R₁ represents H, an alkyl or alkylthio radical containing 1 to 6 carbon atoms;
R₂ and R₃ independently represent H or an alkyl radical containing 1 to 6 carbon atoms;
R₄ represents H₂ or O;
R₅ represents H, or one of the alkyl radicals containing 1 to 6 carbon atoms, alkenyl containing up to 6 carbon atoms, alkynyl containing up to 6 carbon atoms, cycloalkyl, aycloalkylalkyl, the alkyl radical of which contains 1 to 6 carbon atoms, aryl, arylalkyl, the radical alkyl of which contains 1 to 6 carbon atoms, heterocycle or alkyl heterocycle radicals, the alkyl radical of which contains 1 to 6 carbon atoms, these radicals being optionally substituted by radicals chosen from the group comprising an alkyl radical containing 1 to 6 carbon atoms, -O-R₁₀, -S(O)ₘR₁₀ (m representing 0, 1, or 2), -N(R₁₀)(R₁₁), -N-C(O)-R₁₀, -NH-(SO₂)-R₁₀, -CO₂-R₁₀, C(O)-N(R₁₀)(R₁₁), and -(SO₂)-N(R₁₀)(R₁₁) radical;
R₆ and R₇ independently represent H, a -C(O)-NH-CHR₁₃-CO₂R₁₄ radical, or one of the alkyl radicals containing 1 to 6 carbon atoms, aryl, arylalkyl radicals, the alkyl radical of which contains 1 to 6 carbon atoms, heterocycle or alkyl heterocycle radicals, the alkyl radical of which contains I to 6 carbon atoms, these radicals being optionally substituted by radicals chosen from the group comprising the OH, alkyl or alkoxy radicals containing 1 to 6 carbon atoms, N(R₁₀)(R₁₁), COOH, CON(R₁₀)(R₁₁), and halo radicals,
or R₆ and R₇ form together an aryl radical or a heterocycle;
R₈ and R₉ independently represent H or one of the alkyl radicals containing 1 to 6 carbon atoms, aryl, arylalkyl radicals, the alkyl radical of which contains 1 to 6 carbon atoms, heterocycle or alkyl heterocycle radicals, the alkyl radical of which contains 1 to 6 carbon atoms, these radicals being optionally substituted by radicals chosen from the group comprising the OH, alkyl or alkoxy radicals containing 1 to 6 carbon atoms, N(R₁₀)(R₁₁), COOH, CON(R₁₀)(R₁₁) and halo radicals, or R₈ and R₉ form together an aryl radical or a heterocycle;
R₁₀ and R₁₁, independently represent H, an aryl radical or heterocycle, or an alkyl radical containing 1 to 6 carbon atoms, arylalkyl or alkyl heterocycle radical, the alkyl radical of which contains 1 to 6 carbon atoms;
R₁₂ represents NR₉, S, or O;
R₁₃ represents an alkyl radical containing I to 6 carbon atoms optionally substituted by a radical chosen from the alkyl radicals containing 1 to 6 carbon atoms, -OR₁₀, -S(O)ₘR₁₀ (m representing 0, 1, or 2) and -N(R₁₀)(R₁₁) radicals;
R₁₄ represents H or an alkyl radical containing 1 to 6 carbon atoms;
the compound of general formula (I) if appropriate also being able to be in the dimeric form of a disulphide;
or a pharmaceutically acceptable salt of a compound of general formula **(I)** or if appropriate of its dimer;
and at least one other anti-hypertensive agent chosen from the group constituted by the calcium channel inhibitors, conversion enzyme inhibitors, thiazidic diuretics and substitutes, loop diuretics, potassium-sparing diuretics, antialdosterones, beta-blockers, angiotensin receptor antagonists, central anti-hypertensive agents, alpha-blockers and vasodilatory agents, vasopeptidase inhibitors and endothelin antagonists for therapeutic use which is simultaneous, separate or spread out over time, in the treatment of arterial hypertension.

2. Product according to claim 1, **characterized in that** the compound of general formula **(I)** is such that:
X and Y complete a ring with 6 members, X-Y together representing the -CH(R₈)-CH(R₉)- radical;
R₁ represents an alkyl radical containing 1 to 6 carbon atoms;
R₂ and R₃ represent H;
R₄ represents O;
R₅ represents H, or one of the alkyl radicals containing 1 to 6 carbon atoms, cycloalkyl, cycloalkylalkyl, the alkyl radical of which contains 1 to 6 carbon atoms, arylsulphonylalkyl, the alkyl radical of which contains 1 to 6 carbon atoms, aralkoxyalkyl radicals, the alkoxy and alkyl radicals of which each contain 1 to 6 carbon atoms, these radicals being optionally substituted by radicals chosen from the group comprising an alkyl radical containing I to 6 carbon atoms or -O-R₁₀ radical;
R₆ and R₇ independently represent H or an aryl radical optionally substituted by radicals chosen from the group comprising the OH and alkyl or alkoxy radicals containing 1 to 6 carbon atoms;
R₈ and R₉ represent H;
and R₁₀ and R ₁₁, independently represent H or an alkyl radical containing 1 to 6 carbon atoms.

3. Product according to claim 1, **characterized in that** the compound of general formula (I) is one of the following compounds:
- 7-(2-amino-1-oxo-3-thiopropyl)-8-(cyclohexylmethyl)-2-(2-methylphenyl)-5,6,7,8-tetrahydroimidazo[1,2a]pyrazine;
- 7-(2-amino-1-oxo-3-thiopropyl)-8-butyl-2-(2-methoxyphenyl)-5,6,7,8-tetrahydroimidazo[1,2a]pyrazine;
- bis-1,1'-[7-(2-amino-1-oxo-3-thiopropyl)-2-(2-methoxyphenyl)-8-(1-methylpropyl)-5,6,7,8-tetrahydroimidazo[1,2a]pyrazine] disulphide;
- bis-1,1'-[7-(2-amino-1-oxo-3-thiopropyl)-8-(cyclohexylmethyl)-2-(2-methoxyphenyl)-5,6,7,8-tetrahydroimidazo[1,2a]pyrazine disulphide;
- bis-1,1'-7-(2-amino-1-oxo-3-thiopropyl-(2-(1-naphthyl)-8-(2-methylpropyl)-5,6,7,8-tetrahydroimidazo[1,2a]pyrazin-7-yl) disulphide;
- 7-(2-amino-1-oxo-3-thiopropyl)-8-(cyclohexylmethyl)-2-phenyl-5,6,7,8-tetrahydroimidazo[1,2a]pyrazine;
- 7-(2-amino-1-oxo-3-thiopropyl)-2-(2-methoxyphenyl)-8-(phenylmethoxy)methyl-5,6,7,8-tetrahydroimidazo[1,2a]pyrazine;
- 7-(2-amino-1-oxo-3-thiopropyl)-2-(2-methoxyphenyl)-8-(1-phenylmethoxy)ethyl-5,6,7,8-tetrahydroimidazo[1,2a]pyrazine;
- 7-(2-amino-1-oxo-3-thiopropyl)-2-(2-methoxyphenyl)-8-(phenoxyethyl)-5,6,7,8-tetrahydroimidazo[1,2a]pyrazine;
- 7-(2-amino-1-oxo-3-thiopropyl)-2-(2-methoxyphenyl)-8-(phenoxyethyl)-5,6,7,8-tetrahydro-imidazo[1,2a]pyrazine, or its dimeric form;
- and 7-(2-amino-1-oxo-3-thiopropyl)-2-(2-methoxyphenyl)-8-(phenylsulphonylethyl)-5,6,7,8-tetrahydro-imidazo[1,2a]pyrazine;
or a pharmaceutically acceptable salt of one of the latter.

4. Product according to claim 3, **characterized in that** the compound of general formula **(I)** is 7-(2-amino-1-oxo-3-thiopropyl)-8-(cyclohexylmethyl)-2-phenyl-5,6,7,8-tetrahydro-imidazo[1,2a]pyrazine or a pharmaceutically acceptable salt of the latter.

5. Product according to one of claims 1 to 4, **characterized in that** the anti-hypertensive agent combined with the heterotrimeric G protein signal transduction inhibitor is a calcium channel inhibitor.

6. Product according to claim 5, **characterized in that** the anti-hypertensive agent combined with the heterotrimeric G protein signal transduction inhibitor is verapamil.

7. Product according to one of claims 1 to 4, **characterized in that** the anti-hypertensive agent combined with the heterotrimeric G protein signal transduction inhibitor is a conversion enzyme inhibitor.

8. Product according to claim 7, **characterized in that** the anti-hypertensive agent combined with the heterotrimeric G protein signal transduction inhibitor is captopril.

9. Product according to one of the previous claims, **characterized in that** it comprises moreover a second anti-hypertensive agent, the latter being different from the compound of general formula **(I)** and from the anti-hypertensive agent which is already combined with it.

10. Product according to claim 9, **characterized in that** the first anti-hypertensive agent combined with the compound of general formula **(I)** is a loop diuretic and the second is a hyperkalaemiating diuretic.

11. Product according to claim 9, **characterized in that** the first anti-hypertensive agent combined with the compound of general formula **(I)** is a thiazidic or related diuretic and the second is a hyperkalaemiating diuretic.

12. Product according to claim 9, **characterized in that** the first anti-hypertensive agent combined with the compound of general formula **(I)** is a conversion enzyme inhibitor and the second is a thiazidic diuretic.

13. Product according to claim 9, **characterized in that** the first anti-hypertensive agent combined with the compound of general formula **(I)** is an antagonist of angiotensin II receptors and the second is a thiazidic diuretic.

14. Product according to claim 9, **characterized in that** the first anti-hypertensive agent combined with the compound of general formula **(I)** is a beta-blocker and the second is a diuretic.

15. Product according to claim 9, **characterized in that** the first anti-hypertensive agent combined with the compound of general formula **(I)** is a beta-blocker and the second is a calcium channel antagonist.

16. Product according to claim 9, **characterized in that** the first anti-hypertensive agent combined with the heterotrimeric G protein signal transduction inhibitor is a beta-blocker and the second is a vasodilatory agent.

17. Pharmaceutical composition comprising at least one compound of general formula **(I)** as defined in claim 1 and at least one other anti-hypertensive agent chosen from the group constituted by calcium channel inhibitors, conversion enzyme inhibitors, thiazidic diuretics and substitutes, loop diuretics, potassium-sparing diuretics, antialdosterones, beta-blockers, angiotensin receptor antagonists, anti-hypertensive agents, alpha-blockers and vasodilatory agents, vasopeptidase inhibitors and endothelin antagonists with optionally one or more pharmaceutically acceptable excipients.

## Patentansprüche

1. Produkt, umfassend mindestens eine Verbindung der allgemeinen Formel (I) 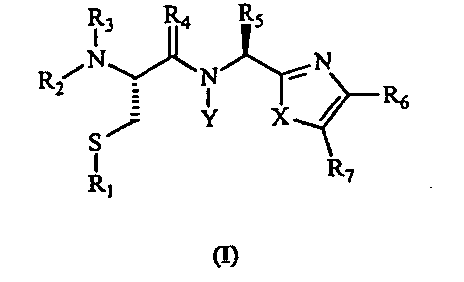 entsprechend den Unterformeln (I_{A}) oder (I_{B}): 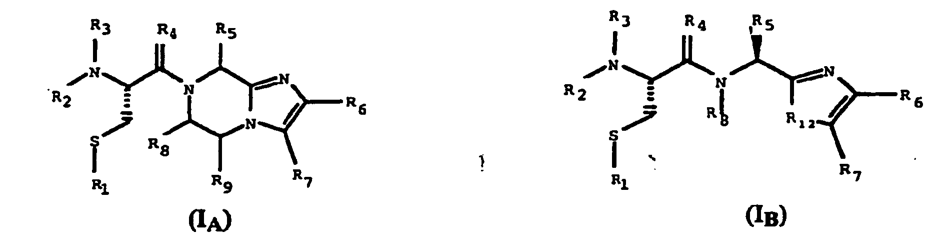 in denen:
X R₁₂ darstellt und Y R₈ darstellt oder X und Y einen Ring mit 6 Gliedern vervollständigen, wobei die Einheit X-Y den Rest -CH(R₈)-CH(R₉)- darstellt;
R₁ H, einen Alkyl- oder Alkylthiorest mit 1 bis 6 Kohlenstoffatomen darstellt;
R₂ und R₃ unabhängig voneinander H oder einen Alkylrest mit 1 bis 6 Kohlenstoffatomen darstellen;
R₄ H₂ oder O darstellt;
R₅ H oder einen der Reste Alkyl mit 1 bis 6 Kohlenstoffatomen, Alkenyl mit bis zu 6 Kohlenstoffatomen, Alkinyl mit bis zu 6 Kohlenstoffatomen, Cycloalkyl, Cycloalkylalkyl, dessen Alkylrest 1 bis 6 Kohlenstoffatome aufweist, Aryl, Arylalkyl, dessen Alkylrest 1 bis 6 Kohlenstoffatome aufweist, Heterocyclus oder Alkylheterocyclus, dessen Alkylrest 1 bis 6 Kohlenstoffatome aufweist, wobei diese Reste ggf. durch Reste substituiert sein können, die aus der Gruppe ausgewählt sind, die aus einem Alkylrest mit 1 bis 6 Kohlenstoffatomen, -O-R₁₀, -S(O)ₘR₁₀ (worin m 0, 1 oder 2 darstellt), -N(R₁₀)(R₁₁), -N-C(O)-R₁₀, -NH-(SO₂)-R₁₀, -CO₂-R₁₀, C(O)-N (R₁₀)(R₁₁) und - (SO₂)-N(R₁₀)(R₁₁) besteht;
R₆ und R₇ unabhängig voneinander H, einen Rest -C(O)-NH-CHR₁₃-CO₂R₁₄ oder einen der Reste Alkyl mit 1 bis 6 Kohlenstoffatomen, Aryl, Arylalkyl, dessen Alkylrest 1 bis 6 Kohlenstoffatome aufweist, Heterocyclus oder Alkylheterocyclus, dessen Alkylrest 1 bis 6 Kohlenstoffatome aufweist, darstellen, wobei diese Reste ggf. durch Reste substituiert sein können, die aus der Gruppe ausgewählt sind, die aus den Resten OH, Alkyl oder Alkoxy mit 1 bis 6 Kohlenstoffatomen, N(R₁₀)(R₁₁), COOH, CON(R₁₀)(R₁₁) und Halogen besteht, oder R₆ und R₇ zusammen einen Arylrest oder einen Heterocyclus bilden;
R₈ und R₉ unabhängig voneinander H oder einen der Reste Alkyl mit 1 bis 6 Kohlenstoffatomen, Aryl, Arylalkyl, dessen Alkylrest 1 bis 6 Kohlenstoffatome aufweist, Heterocyclus oder Alkylheterocyclus, dessen Alkylrest 1 bis 6 Kohlenstoffatome aufweist, darstellen, wobei diese Reste ggf. durch Reste substituiert sein können, die aus der Gruppe ausgewählt sind, die aus den Resten OH, Alkyl oder Alkoxy mit 1 bis 6 Kohlenstoffatomen, N(R₁₀)(R₁₁), COOH, CON(R₁₀)(R₁₁) und Halogen besteht,
oder R₈ und R₉ zusammen einen Arylrest oder einen Heterocyclus bilden;
R₁₀ und R₁₁ unabhängig voneinander H, einen Aryl- oder Heterocyclusrest oder einen Alkylrest mit 1 bis 6 Kohlenstoffatomen, Arylalkyl oder Alkylheterocyclus, dessen Alkylrest 1 bis 6 Kohlenstoffatome aufweist, darstellen;
R₁₂ NR₉, S oder O darstellt;
R₁₃ einen Alkylrest mit 1 bis 6 Kohlenstoffatomen darstellt, der ggf. durch einen Rest substituiert ist, der aus den Resten Alkyl mit 1 bis 6 Kohlenstoffatomen, -OR₁₀, -S(O)ₘR₁₀ (worin m 0, 1 oder 2 darstellt) und -N(R₁₀) (R₁₁) ausgewählt ist;
R₁₄ H oder einen Alkylrest mit 1 bis 6 Kohlenstoffatomen darstellt;
wobei die Verbindung der allgemeinen Formel (I) ggf. auch in Form eines Disulfiddimers vorliegen kann;
oder ein pharmazeutisch annehmbares Salz einer Verbindung der allgemeinen Formel (I) oder ggf. ihres Dimers;
und mindestens ein anderes Antihypertonikum, das aus der Gruppe ausgewählt ist, die aus den Calciumkanalinhibitoren, den Konversionsenzyminhibitoren, den Thiazid-Diuretika und damit verwandten Stoffen, den Schleifendiuretika, den kaliumsparenden Diuretika, den Antialdosteronen, den Betablockern, den Angiotensin-Rezeptor-Antagonisten, den zentralen Antihypertonika, den Alphablockern und Vasodilatoren, den Vasopeptidaseinhibitoren und den Endothelin-Antagonisten besteht, für eine gleichzeitige, getrennte oder zeitlich versetzte therapeutische Verwendung bei der Behandlung der arteriellen Hypertonie.

2. Produkt nach Anspruch 1, **dadurch gekennzeichnet, dass** die Verbindung der allgemeinen Formel (I) so beschaffen ist, dass:
X und Y einen Ring mit 6 Gliedern vervollständigen, wobei die Einheit X-Y den Rest -CH(R₈)-CH(R₉)- darstellt;
R₁ einen Alkylrest mit 1 bis 6 Kohlenstoffatomen darstellt;
R₂ und R₃ H darstellen;
R₄ 0 darstellt;
R₅ H oder einen der Reste Alkyl mit 1 bis 6 Kohlenstoffatomen, Cycloalkyl, Cycloalkylalkyl, dessen Alkylrest 1 bis 6 Kohlenstoffatome aufweist, Arylsulfonylalkyl, dessen Alkylrest 1 bis 6 Kohlenstoffatome aufweist, Aralkoxyalkyl, dessen Alkoxy- und Alkylreste jeweils 1 bis 6 Kohlenstoffatome aufweisen, darstellt, wobei diese Reste ggf. durch Reste substituiert sein können, die aus der Gruppe ausgewählt sind, die aus einem Rest Alkyl mit 1 bis 6 Kohlenstoffatomen oder -O-R₁₀ besteht;
R₆ und R₇ unabhängig voneinander H oder einen Arylrest darstellen, der ggf. durch Reste substituiert ist, die aus der Gruppe ausgewählt sind, die aus den Resten OH und Alkyl oder Alkoxy mit 1 bis 6 Kohlenstoffatomen besteht;
R₈ und R₉ H darstellen;
und R₁₀ und R₁₁ unabhängig voneinander H oder einen Alkylrest mit 1 bis 6 Kohlenstoffatomen darstellen.

3. Produkt nach Anspruch 1, **dadurch gekennzeichnet, dass** die Verbindung der allgemeinen Formel (I) eine der folgenden Verbindungen ist:
- 7-(2-Amino-1-oxo-3-thiopropyl)-8-(cyclohexylmethyl)-2-(2-methylphenyl)-5,6,7,8-tetrahydroimidazo[1,2a]pyrazin;
- 7-(2-Amino-1-oxo-3-thiopropyl)-8-butyl-2-(2-methoxyphenyl)-5,6,7,8-tetrahydroimidazo[1,2a]pyrazin;
- Bis-1,1'-[7-(2-amino-1-oxo-3-thiopropyl)-2-(2-methoxyphenyl)-8-(1-methylpropyl)-5,6,7,8-tetrahydroimidazo[1,2a]-pyrazin]disulfid;
- Bis-1,1'-[7-(2-amino-1-oxo-3-thiopropyl)-8-(cyclohexylmethyl)-2-(2-methoxyphenyl)-5,6,7,8-tetrahydroimidazo-[1,2a]pyrazindisulfid;
- Bis-1,1'-7-(2-amino-1-oxo-3-thiopropyl-(2-(1-naphtyl)-8-(2-methylpropyl)-5,6,7,8-tetrahydroimidazo[1,2a]pyrazin-7-yl-disulfid;
- 7-(2-Amino-1-oxo-3-thiopropyl)-8-(cyclohexylmethyl)-2-phenyl-5,6,7,8-tetrahydroimidazo[1,2a]pyrazin;
- 7-(2-Amino-1-oxo-3-thiopropyl)-2-(2-methoxyphenyl)-8-(phenylmethoxy)methyl-5,6,7,8-tetrahydroimidazo[1,2a]-pyrazin;
- 7-(2-Amino-1-oxo-3-thiopropyl)-2-(2-methoxyphenyl)-8-(1-phenylmethoxy)ethyl-5,6,7,8-tetrahydroimidazo[1,2a]-pyrazin;
- 7-(2-Amino-1-oxo-3-thiopropyl)-2-(2-methoxyphenyl)-8-(phenoxyethyl)-5,6,7,8-tetrahydroimidazo[1,2a]pyrazin;
- 7-(2-Amino-1-oxo-3-thiopropyl)-2-(2-methoxyphenyl)-8-(phenoxyethyl)-5,6,7,8-tetrahydroimidazo[1,2a]pyrazin oder seine Dimerform;
- und 7-(2-Amino-1-oxo-3-thiopropyl)-2-(2-methoxyphenyl)-8-(phenylsulfonylethyl)-5,6,7,8-tetrahydroimidazo[1,2a]-pyrazin;
oder ein pharmazeutisch annehmbares Salz dieser Verbindungen.

4. Produkt nach Anspruch 3, **dadurch gekennzeichnet, dass** die Verbindung der allgemeinen Formel (I) 7-(2-Amino-1-oxo-3-thiopropyl)-8-(cyclohexylmethyl)-2-phenyl-5,6,7,8-tetrahydroimidazo[1,2a]pyrazin oder ein pharmezeutisch annehmbares Salz davon ist.

5. Produkt nach einem der Ansprüche 1 bis 4, **dadurch gekennzeichnet, dass** das mit dem Signaltransduktions-Inhibitor der heterotrimeren G-Proteine kombinierte Antihypertonikum ein Calciumkanalinhibitor ist.

6. Produkt nach Anspruch 5, **dadurch gekennzeichnet, dass** das mit dem Signaltransduktions-Inhibitor der heterotrimeren G-Proteine kombinierte Antihypertonikum Verapamil ist.

7. Produkt nach einem der Ansprüche 1 bis 4, **dadurch gekennzeichnet, dass** das mit dem Signaltransduktions-Inhibitor der heterotrimeren G-Proteine kombinierte Antihypertonikum ein Konversionsenzyminhibitor ist.

8. Produkt nach Anspruch 7, **dadurch gekennzeichnet, dass** das mit dem Signaltransduktions-Inhibitor der heterotrimeren G-Proteine kombinierte Antihypertonikum Captopril ist.

9. Produkt nach einem der vorhergehenden Ansprüche, **dadurch gekennzeichnet, dass** es außerdem ein zweites Antihypertonikum umfasst, das sich von der Verbindung der allgemeinen Formel (I) und von dem mit ihm bereits kombinierten Antihypertonikum unterscheidet.

10. Produkt nach Anspruch 9, **dadurch gekennzeichnet, dass** das erste mit der Verbindung der allgemeinen Formel (I) kombinierte Antihypertonikum ein Schleifendiuretikum und das zweite ein hyperkaliämierendes Diuretikum ist.

11. Produkt nach Anspruch 9, **dadurch gekennzeichnet, dass** das erste mit der Verbindung der allgemeinen Formel (I) kombinierte Antihypertonikum ein Thiazid-Diuretikum oder ein damit verwandter Stoff und das zweite ein hyperkaliämierendes Diuretikum ist.

12. Produkt nach Anspruch 9, **dadurch gekennzeichnet, dass** das erste mit der Verbindung der allgemeinen Formel (I) kombinierte Antihypertonikum ein Konversionsenzyminhibitor und das zweite ein Thiazid-Diuretikum ist.

13. Produkt nach Anspruch 9, **dadurch gekennzeichnet, dass** das erste mit der Verbindung der allgemeinen Formel (I) kombinierte Antihypertonikum ein Angiotensin-II-Rezeptor-Antagonist und das zweite ein Thiazid-Diuretikum ist.

14. Produkt nach Anspruch 9, **dadurch gekennzeichnet, dass** das erste mit der Verbindung der allgemeinen Formel (I) kombinierte Antihypertonikum ein Betablocker und das zweite ein Diuretikum ist.

15. Produkt nach Anspruch 9, **dadurch gekennzeichnet, dass** das erste mit der Verbindung der allgemeinen Formel (I) kombinierte Antihypertonikum ein Betablocker und das zweite ein Calciumkanalantagonist ist.

16. Produkt nach Anspruch 9, **dadurch gekennzeichnet, dass** das erste mit dem Signaltransduktions-Inhibitor der heterotrimeren G-Proteine kombinierte Antihypertonikum ein Betablocker und das zweite ein Vasodilator ist.

17. Pharmazeutische Zusammensetzung, umfassend mindestens eine Verbindung der allgemeinen Formel (I) gemäß Anspruch 1 und mindestens ein anderes Antihypertonikum, das aus der Gruppe ausgewählt ist, die aus den Calciumkanalinhibitoren, den Konversionsenzyminhibitoren, den Thiazid-Diuretika und damit verwandten Stoffen, den Schleifendiuretika, den kaliumsparenden Diuretika, den Antialdosteronen, den Betablockern, den Angiotensin-Rezeptor-Antagonisten, den Antihypertonika, den Alphablockern und Vasodilatoren, den Vasopeptidaseinhibitoren und den Endothelinantagonisten besteht, ggf. mit einem oder mehreren pharmazeutisch annehmbaren Trägerstoffen.
